# EUROPÄISCHE PATENTSCHRIFT

(11) **EP 1 340 484 B1**
(45) Veröffentlichungstag und Bekanntmachung des Hinweises auf die Patenterteilung: **03.08.2005**
(21) Anmeldenummer: 03001326.2
(22) Anmeldetag: 23.01.2003
(51) Int. Cl.: A61K 6/09

(54) **Dentalmaterialien auf der Basis von substituierten Iminooxadiazindion Derivaten**
Dental materials based on substituted imino oxadiazindione derivatives
Matériaux dentaires à base de dérivés d' iminooxadiazindione

(30) Priorität: 27.02.2002 DE 10208396
(43) Veröffentlichungstag der Anmeldung: 03.09.2003
(73) Patentinhaber: Ivoclar Vivadent AG, 9494 Schaan (LI)
(72) Erfinder: Klare, Martin, Dr., 44137 Dortmund (DE); Radl, Andreas, Dr., 9494 Schaan (LI); Rheinberger, Volker, Dr., 9490 Vaduz (LI)
(74) Vertreter: UEXKÜLL & STOLBERG

(56) Entgegenhaltungen:
- EP-A1- 0 798 299
- EP-A1- 1 002 818
- EP-A2- 0 761 670
- EP-A2- 1 222 910
- DE-A- 3 804 154
- US-A- 3 860 556

## Beschreibung

Die vorliegende Erfindung betrifft Dentalmaterialien, die substituierte Iminooxadiazindion-Derivate enthalten.

Lacke und Farben auf der Basis von Isocyanaten sind seit langem bekannt. Diese härten durch Polyaddition eines Diols, wie 1,4-Butylenglykol, an ein Diisocyanat, wie Hexamethylendiisocyanat, unter Ausbildung von Polyurethanen. Ebenso ist die Verwendung von Isocyanaten in Dentalmaterialien bekannt. Hier werden die Isocyanate häufig mit (Meth)acrylaten kombiniert, so daß eine zweistufige Härtung des Materials durch Diisocyanat-Polyaddition und radikalische Polymerisation möglich ist. Auf diese Weise kann das Dentalmaterial nach dem Einbringen in den Mund des Patienten zunächst durch Polyurethanbildung vorgehärtet und im angehärtetem Zustand modelliert und entstandener Überschuß leicht entfernt werden bevor es dann durch radikalische Polymerisation endgehärtet wird.

Es hat sich jedoch gezeigt, daß technisch bedeutsame Diisocyanate, wie z.B. Hexamethylendiisocyanat (HDI), Isophorondiisocyanat (IPDI) und Bis-(4-isocyanatocyclohexyl)-methan (H₁₂MDI), sowohl in monomerer als auch in oligomerer Form, d.h. beispielsweise in Form von Trimeren, häufig temperatur- und feuchtigkeitsempfindlich sind. Außerdem weisen die oligomeren Formen meist hohe Viskositäten auf, die ihre Weiterverarbeitung erschweren. Beispielsweise enthalten Dentalwerkstoffe häufig einen mehr oder weniger hohen Füllstoffanteil, der homogen in das Material eingearbeitet werden muß, um möglichst gleichförmige Eigenschaften zu erzielen. Das Einarbeiten des Füllstoffs wird durch viskose Monomere erschwert. Zudem ist der Füllstoffgehalt bei hoher Viskosität beschränkt, so daß eine Anpassung der Eigenschaften des Werkstoffs an den gewünschten Einsatzzweck nur in einem begrenztem Umfang möglich ist.

Der Erfindung liegt die Aufgabe zu Grunde, Dentalwerkstoffe zur Verfügung zu stellen, die gegenüber Feuchtigkeit unempfindlich sind und deren Füllstoffgehalt exakt den Bedürfnissen entsprechend eingestellt werden kann.

Diese Aufgabe wird durch Dentalmaterialien gelöst, die mindestens ein Iminooxadiazindion-Derivat und mindestens eine Hydroxylverbindung mit mindestens zwei OH-Gruppen enthalten. Unter Iminooxadiazindion-Derivaten werden Derivate des 6-Imino-1,3,5-oxadiazin-2,4-dions verstanden, die mindestens 2, vorzugsweise mindestens 3 freie Isocyanatgruppen aufweisen.

Bevorzugt sind Dentalmaterialien, die mindestens ein Iminooxadiazindion-Derivat mit der Formel 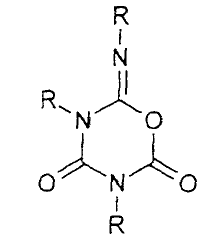 enthalten, in der R ein C₁- bis C₁₆-Alkyl-, C₁- bis C₁₆-Cycloalkyl- und/oder C₁- bis C₁₆-Alkylcycloalkylrest ist, der 1 bis 4, vorzugsweise 1 oder 2, am meisten bevorzugt 1 Isocyanatgruppe aufweist.

Bei den bevorzugten Iminooxadiazindion-Derivaten handelt es sich um asymmetrische Trimere von Diisocyanaten, die dadurch entstehen, daß sich drei Diisocyanatmonomere über jeweils eine Isocyanatgruppe zusammenschließen und die zweite Isocyanatgruppe erhalten bleibt. Die erfindungsgemäß verwendeten substituierten 6-Imino-1,3,5-oxadiazin-2,4-dione werden daher im folgenden der Einfachheit halber auch als Diisocyanat-Trimere bezeichnet.

Unter Alkylcycloalkylreste werden solche Gruppen verstanden, die sowohl Alkyl- als auch Cycloalkylreste enthalten. Bevorzugt sind Alkyl-, Cycloalkyl- und Alkylcyclalkylreste mit 4 bis 14 und insbesondere 6 bis 13 Kohlenstoffatomen. Ganz besonders bevorzugte Iminooxadiazindion-Derivate sind die asymmetrischen Trimere von Hexamethylendiisocyanat. Erfindungsgemäß werden unter Diisocyanat-Trimeren vorzugsweise solche Verbindungen verstanden, die durch drei identische Monomere gebildet werden.

Weitere erfindungsgemäß bevorzugte Iminooxadiazindion-Derivate und deren Herstellung werden in der DE 196 11 849 A2, DE 197 34 048 A2 und von Richter und Mertes, Farbe&Lack 106 (9/2000) Seiten 60 ff. beschrieben.

Die erfindungsgemäß verwendeten Iminooxadiazindion-Derivate lassen sich wie die monomeren Verbindungen durch Isocyanat-Polyaddition härten und zeigen eine mit den symmetrischen Diisocyanat-Trimeren vergleichbare Isocyanat-Reaktivität. Unter den symmetrischen Trimeren werden die entsprechenden Isocyanursäurederivate verstanden, die eine symmetrische Anordnung der Ringatome und Substituenten aufweisen.

Die Iminooxadiazindion-Derivate und insbesondere die asymmetrischen Diisocyanat-Trimere zeichnen sich im Vergleich zu den symmetrischen Trimeren durch eine deutlich erhöhte Feuchtigkeits- und Temperaturstabilität und eine geringere Viskosität aus.

Beispielsweise ist die Viskosität des asymmetrischen Trimers von Hexamethylendiisocyanat (R = -(CH₂)₆-NCO) um den Faktor zwei geringer als die Viskosität des entsprechenden symmetrischen Trimers. Die geringere Viskosität erleichtert das Einarbeiten von Füllstoff in das Material und erlaubt gleichzeitig die Verwendung höherer Füllstoffmengen.

Die erfindungsgemäß bevorzugten Iminooxadiazindion-Derivate weisen eine Viskosität von < 2000 mPas, vorzugsweise von < 1500 mPas und besonders bevorzugt etwa 1000 ± 200 mPas auf. Wenn nicht anders angegeben, handelt es sich um die gemäß DIN EN 3219/A3 gemessene Viskosität.

Die erfindungsgemäßen Dentalmaterialien enthalten vorzugsweise mindestens 5 bis 50 Gew.-%, besonders bevorzugt 7 bis 40 Gew.-% und ganz besonders bevorzugt 9 bis 30 Gew.-% des asymmetrischen Trimers, bezogen auf die Gesamtmasse des Materials.

Als weitere Komponente enthalten die erfindungsgemäßen Dentalwerkstoffe mindestens eine Hydroxylverbindung, die zusammen mit dem Iminooxadiazindion-Derivat durch Diisocyanat-Polyaddition ein Polyurethan bildet.

Als Hydroxylverbindungen eignen sich besonders Polyole. Unter Polyolen werden Verbindungen mit 3 und mehr Hydroxylgruppen verstanden, wobei mit Hydroxylgruppen alkoholische OH-Gruppen gemeint sind. Besonders bevorzugt sind monomere oder polymere aliphatische, alicyclische oder aromatische Verbindung mit 3 bis 6 OH-Gruppen.

Unter den Hydroxylverbindungen mit drei OH-Gruppen sind Trimethylolethan, Trimethylolpropan, Trimethylolbutan, Glycerin und die niedermolekularen Addukte von Ethylen- und/oder Propylenoxid an die genannten Verbindungen als besonders geeignet hervorzuheben.

Bevorzugte Hydroxylverbindungen mit vier OH-Gruppen sind Pentaerythritol und Di-Trimethylolpropan.

Eine bevorzugte Hydroxylverbindung mit sechs OH-Gruppen ist Di-Pentaerythritol.

Weiterhin können auch aliphatische und/oder cycloaliphatische Diole verwendet werden, z.B. Ethylenglycol, Di- und Triethylenglycol, 1,2- und 1,3-Propandiol, Di- und Tripropylenglycol, 1,2- und 1,3- oder 1,4-Butandiol, 1,6 Hexandiol, 2-Methylpentandiol, Neopentylglycol, Cyclohexandiol und Dimethylolcyclohexan.

Bevorzugte polymere Polyole sind Polyesterpolyole, Polyetherpolyole und Polyalkohole, die sowohl Ester- als auch Ethergruppen enthalten, wobei solche Hydroxylverbindungen besonders bevorzugt sind, die ein Äquivalentgewicht von 50 bis 1000, besonders bevorzugt 70 bis 400 pro Hydroxylgruppe aufweisen. Unter den polymeren Polyolen sind weiterhin solche Verbindungen bevorzugt, die eine verzweigte Struktur aufweisen.

Zur Herstellung der erfindungsgemäßen Dentalmaterialien eignen sich besonders Hydroxylverbindungen, die zusätzlich zu den Hydroxylgruppen radikalisch polymerisierbare Gruppen aufweisen und so eine Vernetzung der durch die Diisocyanat-Polyaddition gebildeten Polyurethanketten durch radikalische Polymerisation ermöglichen. Als polymerisierbare Gruppen kommen insbesondere Vinyl-, Methacryl- und/oder Acrylgruppen in Betracht.

Zu den bevorzugten Hydroxylverbindungen mit radikalisch polymerisierbaren Gruppen gehören die Hydroxyethyl(meth)-acrylate, wie z.B. 2-Hydroxyethylacrylat, 2-Hydroxyethylmethacrylat, 2-Hydroxypropyl-methacrylat, 4-Hydroxybutylacrylat und 4-Hydroxybutylmethacrylat.

Als Hydroxylkomponente kann vorteilhaft auch eine Mischung der oben genannten Hydroxylverbindungen verwendet werden, vorzugsweise eine Mischung aus monomeren und polymeren Hydroxylverbindungen. Eine bevorzugte Mischung enthält 28 bis 38 Gew.-%, bevorzugt 30 bis 36 Gew.-% monomere Hydroxylverbindung, insbesondere Trimethylolpropan, und 62 bis 72 Gew.-%, bevorzugt 64 bis 70 Gew.-% polymere Hydroxylverbindung, insbesondere hydroxylgruppenhaltigen Polyester und/oder ester- und ethergruppenhaltigen Polyalkohol, wobei die polymeren Komponenten gemäß einer weiter bevorzugten Ausführungsform eine verzweigte Struktur aufweisen.

Der Gehalt an Hydroxylverbindung in den Dentalmaterialien richtet sich in erster Linie nach der Menge an Iminooxadiazindion-Derivat. Bevorzugt ist ein Gehalt von 4 bis 40 Gew.-%, vorzugsweise 5 bis 20 Gew.-% und insbesondere 6 bis 12 Gew.-%, bezogen auf die Gesamtmasse des Materials.

Dentalmaterialien, die neben dem Iminooxadiazindion-Derivat radikalisch polymerisierbare Hydroxylverbindungen enthalten, können in zwei Stufen gehärtet werden. Vorzugsweise werden die Werkstoffe in einer ersten Stufe durch die Diisocyanat-Polyaddition vorgehärtet. Im vorgehärtetem Zustand sind die Materialien bereits relativ fest und formbeständig, können aber noch modelliert und überschüssiges Material kann leicht entfernt werden. Die Endhärtung erfolgt durch radikalische Polymerisation.

Neben Iminooxadiazindion-Derivat und Hydroxylverbindung enthalten die erfindungsgemäßen Dentalmaterialien vorzugsweise zusätzlich ein oder mehrere radikalisch polymerisierbare Monomere. Diese lassen sich gegebenenfalls mit den radikalisch polymerisierbare Gruppen aufweisenden Hydroxylverbindungen copolymerisieren. Werkstoffe, die sowohl radikalisch polymerisierbares Monomer als auch Hydroxylverbindungen mit radikalisch polymerisierbaren Gruppen enthalten, sind erfindungsgemäß besonders geeignet.

Als radikalisch polymerisierbare Monomere eignen sich insbesondere mono- und polyfunktionelle (Meth)acrylate, d.h. Verbindungen mit einer oder mehreren (Meth)acrylatgruppen. Die nachfolgend genannten Verbindungen sind besonders bevorzugt: 2,2-Bis-[4-(2'-hydroxy-3'-methacryloxy-propoxy)phenylen]propan (Bis-GMA), ethoxyliertes Bisphenol-A-Dimethacrylat, Urethandimethacrylate, Mono-, Di-, Tri- und Tetraethylenglycoldimethacrylate, Neopentylglycoldimeth-acrylate, 1,3-Butandioldimethacrylat, 1,6-Hexandioldimeth-acrylat, 2,2-Bis[4-(2-hydroxy-3-acryloyloxypropan)phenyl]-propan oder 2,2-Bis[4-(acryloyloxy-ethoxy)phenyl]propan. Neben den vorstehend genannten Dimethacrylaten sind auch die entsprechenden Diacrylate bevorzugt. Von den obigen Monomeren sind diejenigen mit zwei oder mehr (Meth)acrylatgruppen besonders bevorzugt, ebenso wie Monomermischungen, die mindestens ein Monomer mit zwei oder mehr (Meth)acrylatgruppen als Vernetzer enthalten.

Die erfindungsgemäßen Dentalmaterialien enthalten vorzugsweise auch einen oder mehrere Füllstoffe. Als Füllstoffe können praktisch alle bekannten und bewährten Füllstoffe eingesetzt werden, die bisher in Dentalmaterialien Verwendung fanden, wobei die Größe der Füllstoffpartikel bevorzugt unter 200 µm, vorzugsweise im Bereich von 10 nm bis 200 µm liegen und der Füllstoff vor der Einarbeitung in die Dentalmaterialien silanisiert werden sollte, bevorzugt mit γ-Methacryloxypropyltrimethoxysilan.

Zu den bevorzugten Füllstoffen zählen insbesondere anorganische Glas-, Glaskeramik- und Keramikpulver mit einer durchschnittlichen Partikelgröße von 0,01 bis 10 µm, bevorzugt 0,1 bis 5 µm, wie z.B. feinteilige Borsilikatgläser, Yb-, Zr- und La- Gläser, Alkali- und Erdalkalisilikate, sowie röntgenopake Füllstoffe, wie z.B. Ytterbiumtrifluorid und Wismutcarbonat, pyrogene Kieselsäure und Fällungskieselsäure, gefüllte und ungefüllte organische Füllstoffe auf der Basis gemahlener Poly(meth)acrylate, Polycarbonate oder Polyepoxide sowie Mischungen der genannten Materialien. Füller auf der Basis gehärteter organischer Materialien werden auch als Isofüller bezeichnet. Die Partikelgröße von Isofüllern liegt vorzugsweise im Bereich von > 10 µm bis 200 µm, insbesondere > 10 µm bis 50 µm. Eine weitere Gruppe bevorzugter Füllstoffe stellen die Zeolithe dar, wobei Zeolithe vom Typ A besonders bevorzugt sind.

Die erfindungsgemäßen Dentalmaterialien enthalten vorzugsweise auch einen Katalysator für die Urethanreaktion und/oder einen Initiator für die radikalische Polymerisation.

Als Katalysatoren für die Urethanreaktion können verschiedene Organozinnverbindungen eingesetzt werden. Bevorzugte Katalysatoren sind Dibutylzinnoxid, Dioctylzinnoxid, Dibutylzinnglycolat, -diacetat, -dilaurat, -maleat, Dilaurylzinndiacetat, Dibutylzinnmaleinatester, Di-n-octylzinnmaleinatester, Dibutylzinnalkylmercaptid, Dibutylzinnmercaptoester, Tributylzinnlaurat und Di-n-octylzinncarboxylate.

Als Initiatoren für die radikalische Polymerisation eignen sich besonders Dibenzoylperoxid (DBPO), Di-p-Chlorbenzoylperoxid, tert.-Butylperoxybenzoat und Cumolhydroperoxid. Ebenso bevorzugt sind Initiatoren für die Photopolymerisation, wie Campherchinon. Weitere geeignete Photoinitiatoren werden in der US 4,746,686 beschrieben. Bevorzugte Initiatoren sind Dibenzoylperoxid und Campherchinon.

Die Initiatoren, wie zum Beispiel die genannten Peroxide, werden vorzugsweise in Kombination mit Polymerisationsakzeleratoren eingesetzt. Bevorzugte Beschleuniger sind tertiäre Amine, wie Triethanolamin, N,N,3,5-Tetramethylanilin, Dimethylamino-benzoesäureester, Dimethyl-p-toluidin oder Dihydroxyethyl-p-toluidin.

Werden die Peroxide allein eingesetzt, werden heißhärtende, in Kombination mit Beschleunigern kalthärtende oder selbsthärtende Materialien erhalten. Durch die Kombination von Photoinitiatoren mit Initiatoren für die Heiß- oder Kalthärtung werden dual härtbare Werkstoffe zugänglich.

Neben den genannten Bestandteilen können die erfindungsgemäßen Dentalwerkstoffe Stabilisatoren und Additive enthalten, wie z.B. Mittel zur Einstellung der Viskosität, Pigmente, Weichmacher und antimikrobielle Zusatzstoffe.

Als Stabilisatoren sind Benzochinon, Hydrochinon, Hydrochinonmonomethylether, 2,6-Di-tert.-butyl-p-kresol (butyliertes Hydroxytoluol, BHT), Phenol und phenolische Verbindungen sowie Chloranil bevorzugt. Die Stabilisatoren sollen eine vorzeitige Polymerisation des Werkstoffs verhindern. Bevorzugter Stabilisator ist 2,6-Di-tert.-butyl-p-kresol.

Die erfindungsgemäßen Dentalmaterialien werden vorzugsweise in Form von zwei Komponenten bereitgestellt, einer ersten und einer zweiten Komponente. Auf diese Weise lassen sich reaktive Bestandteile des Materials, wie z.B. Iminooxadiazindion-Derivat und Hydroxylverbindung oder Initiator und Beschleuniger, auf unterschiedliche Komponenten verteilen. Vorzugsweise enthält die erste Komponente die Hydroxylverbindung und die zweite Komponente das Iminooxadiazindion-Derivat, so daß durch Mischen der Komponenten die Diisocyanat-Polyaddition ausgelöst werden kann. Bei der Verwendung von Initiator/Beschleuniger-Systemen kann auch die radikalische Polymerisation durch das Mischen der Komponenten gestartet werden.

Die erste Komponente (Basenpaste) enthält neben der Hydroxylverbindung soweit vorhanden vorzugsweise auch radikalisch polymerisierbares Monomer, Füllstoff, Katalysator, Stabilisator und Additive.

Gemäß einer besonders bevorzugten Ausführungsform der Erfindung enthält die erste Komponente folgende Bestandteile:
3 bis 85 Gew.-%, vorzugsweise 31 bis 79 Gew.-% und besonders bevorzugt 46 bis 72 Gew.-% Hydroxylverbindung.
15 - 50 Gew.-%, vorzugsweise 20 bis 40 Gew.-%, besonders bevorzugt 25 bis 35 Gew.-% radikalisch polymerisierbares Monomer,
0 - 75 Gew.-%, vorzugsweise 0,2 bis 4 Gew.-%, besonders bevorzugt 0,5 bis 2 Gew.-% Füllstoff,
0 - 10 Gew.-%, vorzugsweise 0,2 bis 4 Gew.-%, besonders bevorzugt 0,5 bis 2 Gew.-% Katalysator für die Diisocyanat-Polyaddition,
0 - 10 Gew.-%, vorzugsweise 1,5 bis 6 Gew.-%, besonders bevorzugt 2 bis 5 Gew.-% Beschleuniger für die radikalische Polymerisation,
0 - 10 Gew.-%, vorzugsweise 0 bis 3 Gew.-%, besonders bevorzugt 0,01 bis 0,15 Gew.-% Stabilisator und
0 - 15 Gew.-%, vorzugsweise 0 bis 12 Gew.-%, besonders bevorzugt 0,01 bis 10 Gew.-% Additiv,
jeweils bezogen auf die Gesamtmasse der ersten Komponente.

Die zweite Komponente (Katalysatorpaste) enthält neben dem Iminooxadiazindion-Derivat soweit vorhanden vorzugsweise auch Füllstoff, Stabilisator, Initiator und Additive.

Gemäß einer besonders bevorzugten Ausführungsform der Erfindung enthält die zweite Komponente folgende Bestandteile:
15 - 100 Gew.-%, vorzugsweise 20 bis 100 Gew.-%, besonders bevorzugt 25 bis 100 Gew.-% und ganz besonders bevorzugt 35 bis 98 Gew.-% Iminooxadiazindion-Derivat,
0 bis 95 Gew.-%, vorzugsweise 0 bis 75 Gew.-% und besonders bevorzugt 5 bis 50 Gew.-% Füllstoff, wobei als Füllstoff vorzugsweise Zeolith und hochdisperse Kieselsäure in den nachfolgend definierten Mengen eingesetzt werden:
0 - 75 Gew.-%, vorzugsweise 0 bis 60 Gew.-%, besonders bevorzugt 0 bis 25 Gew.-% hochdisperse Kieselsäure,
0 - 75 Gew.-%, vorzugsweise 5 bis 50 Gew.-%, besonders bevorzugt 10 bis 25 Gew.-% Zeolith,
0 - 5 Gew.-%, vorzugsweise 0 bis 4,5 Gew.-%, besonders bevorzugt 2 bis 4,5 Gew.-% Initiator für die radikalische Polymerisation, und
0 - 5 Gew.-%, vorzugsweise 0,005 bis 2 Gew.-%, besonders bevorzugt 0,01 bis 0,5 Gew.-% Stabilisator,
jeweils bezogen auf die Gesamtmasse der zweiten Komponente.

Als hochdisperse Kieselsäure eignet sich besonders pyrogene Kieselsäure.

Bei der Herstellung der Iminooxadiazindion-Derivate fallen in der Regel auch symmetrische Trimere und höhere Oligomere an. Da diese ebenfalls reaktive Isocyanatgruppen aufweisen und so bei der Diisocyanat-Polyaddition in das Polymernetzwerk eingebaut werden, müssen sie nicht notwendigerweise von den Iminooxadiazindion-Derivaten abgetrennt werden. Unter höheren Oligomeren werden Verbindungen verstanden, die aus vier und mehr Diisocyanatmonomeren gebildet werden. Die Gesamtmenge an symmetrischen Trimeren und höheren Oligomeren sollte jedoch möglichst gering sein und maximal 70 Gew.-%, vorzugsweise maximal 50 Gew.-%, besonders bevorzugt maximal 20 Gew.-% und ganz besonders bevorzugt maximal 5 Gew.-% betragen, jeweils bezogen auf die Gesamtmasse der zweiten Komponente. Der Gehalt an symmetrischen Trimeren und höheren Oligomeren im Gesamtmaterial kann über das Mischungsverhältnis von erster und zweiter Komponente ermittelt werden. Idealerweise enthalten die Materialien keine symmetrischen Trimere oder höheren Oligomere.

Die erfindungsgemäßen Dentalmaterialien eignen sich vorzugsweise zur Herstellung von Kronen, Brücken und Zementen, insbesondere von provisorischen Kronen und Brücken.

Zweikomponentige Zusammensetzungen für Dentalmaterialien werden bevorzugt als Paste/Paste-Systeme hergestellt, d.h. die Viskosität des oder der Monomere, Füllstoffanteil und Additivgehalt werden so bemessen, daß die jeweilige Komponente eine pastenförmige Konsistenz aufweist.

Die Zusammensetzung der einzelnen Komponenten ist vorzugsweise so bemessen, daß diese in einem Mischungsverhältnis von 10:1 bis 1:1, vorzugsweise 4:1 bis 2:1 und insbesondere 4:1 (bezogen auf das Volumen) eingesetzt werden können. Vorzugsweise wird eine größere Menge an Komponente 1 (Basenpaste) mit einer kleineren Menge an Komponente 2 (Katalysatorpaste) gemischt.

Durch das angegebene Mischungsverhältnis wird sichergestellt, daß sich die erfindungsgemäßen Materialien problemlos in handelsüblichen Mischapparaturen verarbeiten lassen. Hierbei handelt es sich um automatische Mischapparaturen, die üblicherweise einen statischen Mischer und Doppelkartuschen aufweisen. Die Komponenten des Dentalmaterials werden in separaten Kammern der Automix-Kartuschen gelagert. Bei Bedarf werden die Pasten aus den separaten Kammern durch einen statischen Mischer gedrückt. Im Normalfall besteht der statische Mischer aus einer Mischkammer und einer statischen Mischwendel, die bewirkt, daß die einzelnen Pastenströme aus den Pastenkammern vereint, getrennt, wiedervereint und so durchmischt werden. Die Pasten werden in einem vorgegebenen Mischungsverhältnis extrudiert, das durch das Volumenverhältnis der Mischkammern bestimmt wird.

Mischverhältnis und Zusammensetzung der Komponenten werden vorzugsweise so aufeinander abgestimmt, daß der Gehalt an asymmetrischem Trimer in der Gesamtzusammensetzung in den oben angegebenen Bereichen liegt, d.h. mindestens 5 Gew.-%, vorzugsweise 5 bis 50 Gew.-%, besonders bevorzugt 7 bis 40 Gew.-% und ganz besonders bevorzugt 9 bis 30 Gew.-% beträgt.

Im folgenden wird die Erfindung anhand von Ausführungsbeispielen näher erläutert.

### Beispiele

### Beispiel 1: Vergleich der Feuchtigkeitsempfindlichkeit von symmetrischen und asymmetrischen Diisocyanat-Trimeren

Das asymmetrische Hexamethylendiisocyanat- (HDI-) Trimer 3,5-Bis(6-isocyanato-hexyl)-6-[(6-isocyanatohexyl)imino]-1,3,5-oxadiazin-2,4-dion (Desmodur® VP LS2294, Bayer AG) und das symmetrische Isocyanurat-Trimer von HDI (Desmodur® N 3300, Bayer AG) wurden getrennt jeweils in Gebinden mit 10 ml Inhalt bei 37°C in wasserdampfgesättigter Atmosphäre gelagert und die Dicke des sich durch Reaktion der Isocyanatderivate mit Wasser bildenden Films über einen Zeitraum von einer Woche gemessen.

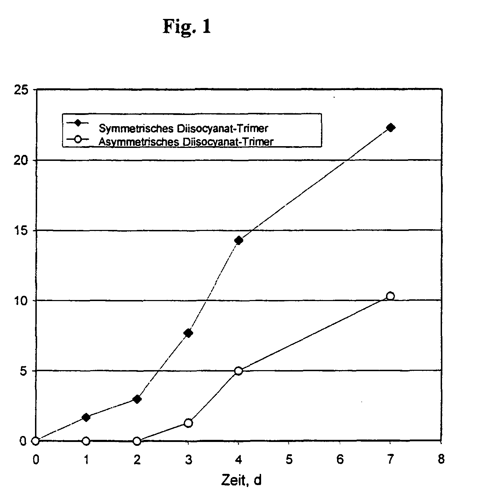

In Figur 1 ist die Schichtdicke in Abhängigkeit von der Zeit graphisch dargestellt. Es ist zu erkennen, daß die Schichtdicke des asymmetrischen Diisocyanat-Trimers (Desmodur® VP LS2294) deutlich langsamer zunimmt als die Schichtdicke des symmetrischen Trimers (Desmodur® N 3300). Die Hautbildung setzt bei dem asymmetrischen Diisocyanat-Trimer unter den gewählten Bedingungen erst nach 3 Tagen spürbar ein und beträgt nach einer Woche weniger als die Hälfte der symmetrischen Variante.

### Beispiel 2: Selbsthärtendes Material zur Herstellung von Kronen und Brücken

Es wurde ein zweikomponentiges Material mit der folgenden Zusammensetzung hergestellt:

| **Basenpaste:** | |
|---|---|
| **Bestandteil** | **Anteil** |
| Monomermischung¹ | 27,50 Gew.-% |
| Hydroxylkomponente² | 9,60 Gew.-% |
| Füllstoff | |
| Bariumglas, silanisiert³ | 30,30 Gew.-% |
| Isofüller⁴ | 16,00 Gew.-% |
| pyrogene Kieselsäure⁵ | 1,90 Gew.-% |
| Katalysator (Di-n-octylzinncarboxylat) | 0,75 Gew.-% |
| Stabilisator (BHT) | 0,05 Gew.-% |
| Polymerisationsakzelerator (Dihydroxyethyl-p-toluidin) | 4,40 Gew.-% |
| Zeolithpaste⁶ | 9,50 Gew.-% |

| | |
|---|---|
| ¹ 10 Gew.-% Bis-GMA, 65,5 Gew.-% Urethandimethacrylat, 24,5 Gew.-% Triethylenglycoldimethacrylat, bezogen auf die Masse der Monomerkomponente | |
| ² Polyalkoholmischung mit 32,8 Gew.-% Trimethylolpropan, 60,5 Gew.-% verzweigtem Polyalkohol mit Ester- und Ethergruppen (Hydroxylgehalt nach DIN 53402 7.1 ± 0,5; Desmophen 1145, Bayer AG) und 6,7 Gew.-% verzweigtem, hydroxylgruppenhaltigem Polyester (Hydroxylgehalt nach DIN 53402 8,6 ± 0,3; Desmophen 800, Bayer AG), bezogen auf die Masse der Hydroxylkomponente | |
| ³ Partikelgröße 5,5 µm ± 2 µm | |
| ⁴ feinteiliges Polymerisat auf der Basis von 70 Gew.-% pyrogener Kieselsäure (BET-Oberfläche 50 m²/g, durchschnittliche Partikelgröße 40 nm), 23 Gew.-% 7,7(9),9-Trimethyl-4,13-dioxo-3,14-dioxa-5,12-diazohexadecan-1,16-diyl-dimethacrylat, 6,4 Gew.-% 1,10-Decandioldimethacrylat und 0,6 Gew.-% Dibenzoxylperoxid, bezogen auf die Masse des Isofüllers | |
| ⁵ BET-Oberfläche 140 m²/g, Primärteilchengröße 10-30 nm, Partikelgröße der Agglomerate 10-100 µm, modifiziert mit -OSi(CH₃)₃ (HDK 2000) | |
| ⁶ Mischung aus 50 Gew.-% Natriumaluminosilikat, 14 Gew.-% pyrogener Kieselsäure, 3,6 Gew.-% Bis-GMA, 23,4 Gew.-% Urethandimethacrylat und 9 Gew.-% Triethylenglykoldimethacrylat, bezogen auf die Masse der Zeolithpaste | |

| **Katalysatorpaste:** | |
|---|---|
| **Bestandteil** | **Anteil** |
| asymmetrisches Trimer^{*}) | 95,90 Gew.-% |
| Dibenzoylperoxid | 4,00 Gew.-% |
| Stabilisator (BHT) | 0,10 Gew.-% |

| | |
|---|---|
| *) 3,5-Bis(6-isocyanato-hexyl)-6-[(6-isocyanatohexyl)imino]-1,3,5-oxadiazin-2,4-dion (Desmodur® VP LS 2294) | |

Basenpaste und Katalysatorpaste wurden im Verhältnis von 4:1 miteinander gemischt. Sowohl die Diisocyanat-Polyaddition als auch die radikalische Polymerisation werden durch das Mischen der Komponenten ausgelöst, d.h. es handelt sich um ein Material, das vollständig selbsthärtend ist.

Nach dem Härten wies das Material einen E-Modul von 1.856 MPa und eine Biegefestigkeit von 66 MPa auf. Beide Werte liegen innerhalb der für kommerzielle provisorische Dentalmaterialien üblichen Bereiche (1.600 bis 3.200 MPa für den E-Modul und 60 bis 85 MPa für die Biegefestigkeit). Soweit nicht anders angegeben wurden E-Modul und Biegefestigkeit in diesem und allen anderen Beispielen gemäß EN ISO 4049 ermittelt.

### Beispiel 3: Selbst- und lichthärtendes Material zur Herstellung von Kronen und Brücken

Es wurde ein zweikomponentiges Material mit der folgenden Zusammensetzung hergestellt. Soweit nicht anders angegeben wurden die gleichen Materialien wie in Beispiel 2 verwendet.

| **Basenpaste:** | |
|---|---|
| **Bestandteil** | **Anteil** |
| Monomermischung¹ | 29,60 Gew.-% |

| Füllstoff | |
|---|---|
| Bariumglas, silanisiert | 32,50 Gew.-% |
| Isofüller | 15,95 Gew.-% |
| pyrogene Kieselsäure | 1,90 Gew.-% |
| Hydroxylkomponente | 9,65 Gew.-% |
| Zeolithpaste | 9,50 Gew.-% |
| Zinn-Katalysator | 0,75 Gew.-% |
| Pigmente (TiO₂) | 0,10 Gew.-% |
| Additive² | 0,05 Gew.-% |

| | |
|---|---|
| ¹ Mischung aus Beispiel 2 + BHT, Campherchinon, Ethyl-p-Dimethylaminobenzoat | |
| ² Weichmacher (Dibutylphthalat), Antimikrobielle Zusätze (Triclosan, Chlorhexidin), Entschäumer (Methyl-Polysiloxane) | |

| **Katalysatorpaste:** | |
|---|---|
| **Bestandteil** | **Anteil** |
| asymmetrisches Trimer^{*}) | 100 Gew.-% |

| | |
|---|---|
| *) 3,5-Bis(6-isocyanato-hexyl)-6-[(6-isocyanato-hexyl) imino] -1,3,5-oxadiazin-2,4-dion (Desmodur® VP LS 2294) | |

Basenpaste und Katalysatorpaste wurden im Verhältnis von 4:1 miteinander gemischt. Die Diisocyanat-Polyaddition wird durch das Mischen der Komponenten ausgelöst, die radikalische Polymerisation wird durch Licht initiiert.

Nach dem vollständigen Härten wies das Material einen E-Modul von 4.034 MPa und eine Biegefestigkeit von 111 MPa auf. Beide Werte übertreffen die bei kommerziellen provisorischen Dentalmaterialien üblichen Werte deutlich.

### Beispiel 4: Einfluß unterschiedlicher Sn-Organyle auf die Diisocyanat-Polyaddition

Zur Bestimmung des Einflusses des Katalysators auf die Geschwindigkeit der Diisocyanat-Polyaddition wurden Dentalmaterialien auf der Basis des asymmetrischen Diisocyanat-Trimers von HDI (Desmodur® VP LS2294, Bayer AG) hergestellt. Als Basenpaste wurde die in Beispiel 3 beschriebene Zusammensetzung verwendet, wobei die unten angegebenen Katalysatoren zur Herstellung der Paste eingesetzt wurden, als Katalysatorpaste dienten jeweils die reinen Diisocyanat-Trimere. Basenpaste und Katalysatorpaste wurden im Verhältnis von 4:1 miteinander gemischt.

Zur Herstellung der Basenpaste verwendete Katalysatoren:

| **Bezeichnung** | **Katalysator** |
|---|---|
| Kat. 1 | Dibutylzinnmercaptid |
| Kat. 2 | Dibutylzinndilaurat |
| Kat. 3 | Di-n-butylzinndilaurat |
| Kat. 4 | Di-n-octylzinndilaurat |
| Kat. 5 | Di-n-octylzinnmaleinatester |
| Kat. 6 | Di-n-octylzinncarboxylat |

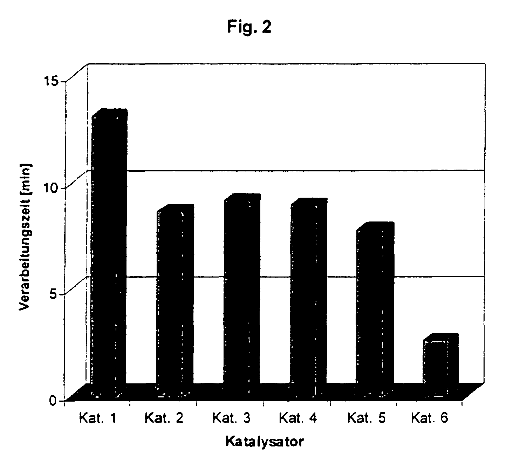

Die zur Herstellung der Materialien verwendete Katalysatormenge war in allen Fällen gleich. Für jedes Material wurde mittels eines Rheometers anhand der Tangentenmethode die zur Verfügung stehende Verarbeitungszeit gemessen (Viskosität nach DIN 53019/1). Die erzielten Ergebnisse sind in Figur 2 dargestellt.

Die gefundenen Ergebnisse zeigen, daß die Verarbeitungszeit stark von der Art des Katalysators abhängt. Durch die Wahl des Katalysators kann somit die Verarbeitungszeit der Dentalmaterialien an die jeweiligen Erfordernisse angepaßt werden.

### Beispiel 5: Selbst- und lichthärtendes Material zur Herstellung von provisorischen Kronen und Brücken

Es wurde ein zweikomponentiges Material mit der folgenden Zusammensetzung hergestellt. Soweit nicht anders angegeben wurden die gleichen Materialien wie in Beispiel 2 verwendet.

| **Basenpaste:** | |
|---|---|
| **Bestandteil** | **Anteil** |
| Monomermischung (wie Beispiel 3) | 29,60 Gew.-% |

| Füllstoff | |
|---|---|
| Bariumglas, silanisiert | 32,50 Gew.-% |
| Isofüller | 15,95 Gew.-% |
| pyrogene Kieselsäure (HDK 2000) | 1,90 Gew.-% |
| Zeolithpaste | 9,50 Gew.-% |
| Hydroxylverbindung | 9,65 Gew.-% |
| Zinn-Katalysator | 0,75 Gew.-% |
| Pigmente | 0,10 Gew.-% |
| Additive | 0,05 Gew.-% |

| **Katalysatorpaste :** | |
|---|---|
| **Bestandteil** | **Anteil** |
| asymmetrisches Trimer*) | 50,0 Gew.-% |
| Zeolithpaste | 50,0 Gew.-% |

| | |
|---|---|
| *) 3,5-Bis(6-isocyanato-hexyl)-6-[(6-isocyanato-hexyl)imino]-1,3,5-oxadiazin-2,4-dion (Desmodur® VP LS 2294) | |

Basenpaste und Katalysatorpaste wurden im Verhältnis von 1:1 miteinander gemischt. Die Diisocyanat-Polyaddition wird durch das Mischen der Komponenten ausgelöst, die radikalische Polymerisation wird durch Licht initiiert.

Nach dem vollständigen Härten wies das Material einen E-Modul von 4.691 MPa und eine Biegefestigkeit von 79 MPa auf. Beide Werte übertreffen die bei kommerziellen provisorischen Dentalmaterialien üblichen Werte deutlich.

### Beispiel 6: Selbst- und lichthärtendes Material zur Herstellung von provisorischen Kronen und Brücken

Es wurde ein zweikomponentiges Material mit der folgenden Zusammensetzung hergestellt. Soweit nicht anders angegeben wurden die gleichen Materialien wie in Beispiel 2 verwendet.

| **Basenpaste:** | |
|---|---|
| **Bestandteil** | **Anteil** |
| Monomermischung (wie Beispiel 3) | 29,60 Gew.-% |

| Füllstoff | |
|---|---|
| Bariumglas, silanisiert | 32,50 Gew.-% |
| Isofüller | 15,95 Gew.-% |
| pyrogene Kieselsäure (HDK 2000) | 1,90 Gew.-% |
| Zeolithpaste | 9,50 Gew.-% |
| Hydroxylverbindung | 9,65 Gew.-% |
| Zinn-Katalysator | 0,75 Gew.-% |
| Pigmente | 0,10 Gew.-% |
| Additive | 0,05 Gew.-% |

| **Katalysatorpaste:** | |
|---|---|
| **Bestandteil** | **Anteil** |
| asymmetrisches Trimer^{*}) | 66,0 Gew.-% |
| Zeolithpaste | 34,0 Gew.-% |

| | |
|---|---|
| *) 3,5-Bis(6-isocyanato-hexyl)-6-[(6-isocyanato-hexyl)imino]-1,3,5-oxadiazin-2,4-dion (Desmodur® VP LS 2294) | |

Basenpaste und Katalysatorpaste wurden im Verhältnis von 2:1 miteinander gemischt. Die Diisocyanat-Polyaddition wird durch das Mischen der Komponenten ausgelöst, die radikalische Polymerisation wird durch Licht initiiert.

Nach dem vollständigen Härten wies das Material einen E-Modul von 3.973 MPa und eine Biegefestigkeit von 63 MPa auf. Der E-Modul übertrifft die bei kommerziellen provisorischen Dentalmaterialien üblichen Werte deutlich, die Biegefestigkeit liegt in dem üblichen Bereich.

### Beispiel 7: Selbsthärtender Dentalzement

Es wurde ein zweikomponentiges Material mit der folgenden Zusammensetzung hergestellt. Soweit nicht anders angegeben wurden die gleichen Materialien wie in Beispiel 2 verwendet.

| **Basenpaste:** | |
|---|---|
| **Bestandteil** | **Anteil** |
| Monomermischung (Mischung aus Beispiel 2 + BHT) | 37,20 Gew.-% |

| Füllstoff | |
|---|---|
| Bariumglas, silanisiert | 24,40 Gew.-% |
| pyrogene Kieselsäure, silanisiert | 8,60 Gew.-% |
| Ytterbiumtrifluorid (röntgenopaker Füllstoff) | 9,10 Gew.-% |
| Zeolithpaste | 8,50 Gew.-% |
| Hydroxylverbindung | 8,55 Gew.-% |
| Polymerisationsakzelerator (Dihydroxyethyl-p-toluidin) | 2,95 Gew.-% |
| Zinn-Katalysator | 0,65 Gew.-% |
| Additive | 0,05 Gew.-% |

| **Katalysatorpaste:** | |
|---|---|
| **Bestandteil** | **Anteil** |
| asymmetrisches Trimer*) | 48,9 Gew.-% |
| Zeolithpaste | 50,0 Gew.-% |
| Dibenzoylperoxid | 1,0 Gew.-% |
| Stabilisator (BHT) | 0,1 Gew.-% |

| | |
|---|---|
| *) 3,5-Bis(6-isocyanato-hexyl)-6-[(6-isocyanato-hexyl)imino]-1,3,5-oxadiazin-2,4-dion (Desmodur® VP LS 2294) | |

Basenpaste und Katalysatorpaste wurden im Verhältnis von 1:1 miteinander gemischt. Sowohl die Diisocyanat-Polyaddition als auch die radikalische Polymerisation werden durch das Mischen der Komponenten ausgelöst, d.h. es handelt sich um ein Material, das vollständig selbsthärtend ist.

Nach dem vollständigen Härten wies das Material eine Druckfestigkeit von 120 MPa auf. Dieser Wert übertrifft die bei kommerziellen provisorischen Dentalmaterialien üblichen Werte (60 bis 85 MPa) deutlich. Wenn nicht anders angegeben wurde die Druckfestigkeit in diesem und in allen anderen Beispielen gemäß EN 29917 mit einer Universalprüfmaschine der Firma Zwick ermittelt.

### Beispiel 8: Selbst- und lichthärtender Dentalzement

Es wurde ein zweikomponentiges Material mit der folgenden Zusammensetzung hergestellt. Soweit nicht anders angegeben wurden die gleichen Materialien wie in Beispiel 2 verwendet.

| **Basenpaste:** | |
|---|---|
| **Bestandteil** | **Anteil** |
| Monomermischung | 40,85 Gew.-% |

| Füllstoff | |
|---|---|
| Bariumglasfüller, silanisiert | 22,20 Gew.-% |
| pyrogene Kieselsäure, silanisiert | 9,80 Gew.-% |
| Ytterbiumtrifluorid | 9,10 Gew.-% |
| Zeolithpaste | 8,90 Gew.-% |
| Hydroxylverbindung | 8,50 Gew.-% |
| Zinn-Katalysator | 0,65 Gew.-% |

| **Katalysatorpaste :** | |
|---|---|
| **Bestandteil** | **Anteil** |
| asymmetrisches Trimer*) | 50,0 Gew.-% |
| Zeolithpaste | 50,0 Gew.-% |

| | |
|---|---|
| *) 3,5-Bis(6-isocyanato-hexyl)-6-[(6-isocyanato-hexyl)imino]-1,3,5-oxadiazin-2,4-dion (Desmodur® VP LS 2294) | |

Basenpaste und Katalysatorpaste wurden im Verhältnis von 1:1 miteinander gemischt. Die Diisocyanat-Polyaddition wird durch das Mischen der Komponenten ausgelöst, die radikalische Polymerisation wird durch Licht initiiert.

Nach dem vollständigen Härten wies das Material eine Druckfestigkeit von 89 MPa auf. Dieser Wert übertrifft die bei kommerziellen provisorischen Dentalmaterialien üblichen Werte.

## Patentansprüche

1. Dentalmaterial enthaltend mindestens ein Iminooxadiazindion-Derivat mit mindestens zwei freien Isocyanatgruppen und mindestens eine Hydroxylverbindung mit mindestens zwei OH-Gruppen.

2. Dentalmaterial nach Anspruch 1, **dadurch gekennzeichnet, daß** es ein Iminooxadiazindion-Derivat mit der Formel 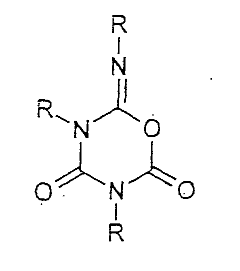 enthält, in der R ein C₁- bis C₁₂-Alkylrest ist, der mindestens eine Isocyanatgruppe aufweist.

3. Dentalmaterial nach Anspruch 1 oder 2, **dadurch gekennzeichnet, daß** es 5 bis 50 Gew.-% Iminooxadiazindion-Derivat enthält.

4. Dentalmaterial nach einem der Ansprüche 1 bis 3, **dadurch gekennzeichnet, daß** es als Hydroxylverbindung eine monomere oder polymere aliphatische, alicyclische oder aromatische Verbindung mit 2 bis 6 OH-Gruppen enthält.

5. Dentalmaterial nach Anspruch 4, **dadurch gekennzeichnet, daß** die Hydroxylverbindung zusätzlich radikalisch polymerisierbare Gruppen aufweist.

6. Dentalmaterial nach Anspruch 5, **dadurch gekennzeichnet, daß** die Hydroxylverbindung mindestens eine Vinyl-, Methacryl- und/oder Acrylgruppe aufweist.

7. Dentalmaterial nach einem der Ansprüche 1 bis 6, **dadurch gekennzeichnet, das** es zusätzlich eines oder mehrere radikalisch polymerisierbare Monomere enthält.

8. Dentalmaterial nach einem der Ansprüche 1 bs 7, **dadurch gekennzeichnet, daß** es einen Katalysator für die Urethanreaktion und/oder einen Initiator für die radikalische Polymerisation enthält.

9. Dentalmaterial nach einem der Ansprüche 1 bis 8, **dadurch gekennzeichnet, daß** es eine erste und eine zweite Komponente enthält, wobei die erste Komponente eine Hydroxylverbindung und die zweite Komponente ein Iminooxadiazindion-Derivat enthält.

10. Dentalmaterial nach Anspruch 9, **dadurch gekennzeichnet, daß** die erste Komponente
| | |
|---|---|
| 3 - 85 Gew.-% | Hydroxylverbindung, |
| 15 - 50 Gew.-% | radikalisch polymerisierbares Monomer, |
| 0 - 75 Gew.-% | Füllstoff, |
| 0 - 10 Gew.-% | Katalysator für die Diisocyanat-Polyaddition, |
| 0 - 10 Gew.-% | Beschleuniger für die radikalische Polymerisation, |
| 0 - 10 Gew.-% | Stabilisator und |
| 0 - 15 Gew.-% | Additive |
enthält, jeweils bezogen auf die Gesamtmasse der ersten Komponente,
und die zweite Komponente
| | |
|---|---|
| 15 - 100 Gew.-% | Iminooxadiazindion-Derivat, |
| 0 - 95 Gew.-% | Füllstoff, |
| 0 - 5 Gew.-% | Initiator für die radikalische Polymerisation, und |
| 0 - 5 Gew.-% | Stabilisator |
enthält, jeweils bezogen auf die Gesamtmasse der zweiten Komponente.

11. Verwendung eines Iminooxadiazindion-Derivats mit mindestens zwei freien Isocyanatgruppen als Dentalmaterial oder zur Herstellung von Dentalmaterialien.

12. Verwendung nach Anspruch 10 zur Herstellung von Kronen, Brücken und Zementen.

## Claims

1. Dental material comprising at least one iminooxadiazine dione derivative with at least two free isocyanate groups and at least one hydroxyl compound with at least two OH groups.

2. Dental material according to Claim 1, **characterised in that** it comprises an iminooxadiazine dione derivative with the Formula 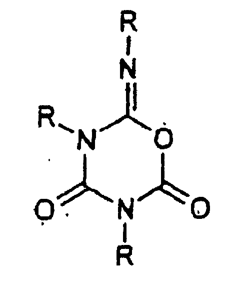 in which R is a C₁ to C₁₂ alkyl radical which contains at least one isocyanate group.

3. Dental material according to Claim 1 or 2, **characterised in that** it comprises 5 to 50 wt.% iminooxadiazine dione derivative.

4. Dental material according to one of Claims 1 to 3, **characterised in that** it comprises a monomeric or polymeric aliphatic, alicyclic or aromatic compound with 2 to 6 OH groups as the hydroxyl compound.

5. Dental material according to Claim 4, **characterised in that** the hydroxyl compound additionally contains radically polymerisable groups.

6. Dental material according to Claim 5, **characterised in that** the hydroxyl compound contains at least one vinyl-, methacrylic- and/or acrylic group.

7. Dental material according to one of Claims 1 to 6, **characterised in that** it further comprises one or more radically polymerisable monomer.

8. Dental material according to one of Claims 1 to 7, **characterised in that** it comprises a urethane reaction catalyst and/or a radical polymerisation initiator.

9. Dental material according to one of Claims 1 to 8, **characterised in that** it comprises a first and a second component, the first component comprising a hydroxyl compound and the second component comprising an iminooxadiazine dione derivative.

10. Dental material according to Claim 9, **characterised in that** the first component comprises
| | |
|---|---|
| 3 - 85 wt.% | hydroxyl compound, |
| 15 - 50 wt.% | radically polymerisable monomer, |
| 0 - 75 wt.% | filler, |
| 0 - 10 wt.% | catalyst for the diisocyanate polyaddition, |
| 0 - 10 wt.% | accelerator for the radical polymerisation, |
| 0 - 10 wt.% | stabiliser and |
| 0 - 15 wt.% | additives |
each based on the total weight of the first component,
and the second component comprises
| | |
|---|---|
| 15 - 100 wt.% | iminooxadiazine dione derivative, |
| 0 - 95 wt.% | filler, |
| 0 - 5 wt.% | initiator for the radical polymerisation, and |
| 0 - 5 wt.% | stabiliser |
each based on the total weight of the second component.

11. Use of an iminooxadiazine dione derivative with at least two free isocyanate groups as dental material or for the manufacture of dental materials.

12. Use according to Claim 10 for the manufacture of crowns, bridges and cements.

## Revendications

1. Matériau dentaire comprenant au moins un dérivé d'iminooxadiazinedione avec au moins deux groupes isocyanates libres et au moins un composé hydroxylé avec au moins deux groupes oH.

2. Matériau dentaire selon la revendication 1, **caractérisé en ce qu'**il contient un dérivé d'iminooxadiazinedione selon la formule 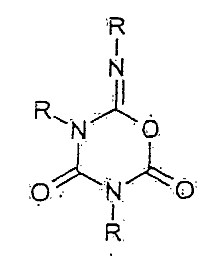 dans laquelle R est un alkyle C₁ à C₁₂, portant au moins un groupe isocyanate.

3. Matériau dentaire selon la revendication 1 ou 2, **caractérisé en ce qu'**il contient 5 à 50 % en poids de dérivé d'iminooxadiazinedione.

4. Matériau dentaire selon l'une quelconque des revendications 1 à 3, **caractérisé en ce qu'**il contient comme composé hydroxylé un composé monomère ou polymère aliphatique, alicyclique ou aromatique avec 2 à 6 groupes OH.

5. Matériau dentaire selon la revendication 4, **caractérisé en ce que** le composé hydroxylé comprend en plus des groupes polymérisables par voie radicalaire.

6. Matériau dentaire selon la revendication 5, **caractérisé en ce que** le composé hydroxylé comprend au moins un groupe vinyle, méthacryle, et/ou acryle.

7. Matériau dentaire selon l'une quelconque des revendications 1 à 6, **caractérisé en ce qu'**il contient en plus un ou plusieurs monomères polymérisables par voie radicalaire.

8. Matériau dentaire selon l'une quelconque des revendications 1 à 7, **caractérisé en ce qu'**il contient un catalyseur pour la réaction uréthanique et/ou un initiateur pour la polymérisation radicalaire.

9. Matériau dentaire selon l'une quelconque des revendications 1 à 8, **caractérisé en ce qu'**il contient un premier et un deuxième composant, le premier composant contenant un composé hydroxylé et le deuxième composant un dérivé d'iminooxadiazinedione.

10. Matériau dentaire selon la revendication 9, **caractérisé en ce que** le premier composant contient
| | |
|---|---|
| 3 à 85 % en poids | de composé hydroxylé, |
| 15 à 50 % en poids | de monomère polymérisable par voie radicalaire, |
| 0 à 75 % en poids | de matière de charge, |
| 0 à 10 % en poids | de catalyseur pour la polyaddition au diisocyanate, |
| 0 à 10 % en poids | d'accélérateur pour la polymérisation radicalaire, |
| 0 à 10 % en poids | de stabilisant, et |
| 0 à 15 % en poids | d'additifs, |
rapporté chaque fois à la masse totale du premier composant,
et **en ce que** le deuxième composant contient
| | |
|---|---|
| 15 à 100 % en poids | de dérivé d'iminooxadiazinedione, |
| 0 à 95 % en poids | de matière de charge, |
| 0 à 5 % en poids | d'initiateur pour la polymérisation radicalaire, et |
| 0 à 5 % en poids | de stabilisant, |
rapporté chaque fois à la masse totale du deuxième composant.

11. Utilisation d'un dérivé d'iminooxadiazinedione avec au moins deux groupes isocyanates libres, comme matériau dentaire ou destiné à la fabrication de matériaux dentaires.

12. Utilisation selon la revendication 10 pour la fabrication de couronnes, de bridges et de ciments.
